# EUROPÄISCHE PATENTSCHRIFT

(11) **EP 2 552 393 B1**
(45) Veröffentlichungstag und Bekanntmachung des Hinweises auf die Patenterteilung: **04.03.2015**
(21) Anmeldenummer: 11700956.3
(22) Anmeldetag: 27.01.2011
(51) Int. Cl.: A61K 8/49, A61Q 5/08, A61K 8/31, A61Q 5/10

(54) **AUFHELLMITTEL MIT LIPOPHILEM ACYLPYRIDINIUM-KOMPLEX**
LIGHTENER WITH LIPOPHILIC ACYLPYRIDINIUM COMPLEX
AGENT ÉCLAIRCISSANT COMPRENANT UN COMPLEXE D'ACYLPYRIDINIUM LIPOPHILE

(30) Priorität: 29.03.2010 DE 102010003394
(43) Veröffentlichungstag der Anmeldung: 06.02.2013
(73) Patentinhaber: Henkel AG & Co. KGaA, 40589 Düsseldorf (DE)
(72) Erfinder: MANNECK, Hartmut, 23860 Klein Wesenberg (DE); KLEEN, Astrid, 20457 Hamburg (DE); BREWER, Marie-Lisa, 22761 Hamburg (DE)
(86) Internationale Anmeldenummer: PCT/EP2011/051115
(87) Internationale Veröffentlichungsnummer: WO 2011/124401

(56) Entgegenhaltungen:
- WO-A1-2005/055966
- WO-A1-2009/135700
- WO-A1-2010/022996
- WO-A2-2010/029006
- WO-A2-2010/054981
- WO-A2-2010/066720
- WO-A2-2010/072512
- WO-A2-2010/130513
- DE-A1-102007 047 685

## Beschreibung

Die vorliegende Erfindung betrifft Mittel zum Aufhellen keratinischer Fasern, insbesondere menschlicher Haare, enthaltend einen lipophilen Acylpyridinium-Komplex aus kationischen Acylpyridiniumderivate und Paraffinöl.

Die Veränderung von Form und Farbe der Haare stellt einen wichtigen Bereich der modernen Kosmetik dar. Neben der Färbung ist das Aufhellen der eigenen Haarfarbe bzw. das Blondieren der ganz spezielle Wunsch vieler Verbraucher, da eine blonde Haarfarbe als attraktiv und in modischer Hinsicht erstrebenswert betrachtet wird. Für diesen Zweck sind im Markt verschiedene Blondiermittel mit unterschiedlicher Blondierleistung erhältlich. Die Grundlagen der Blondierverfahren sind dem Fachmann bekannt und können in einschlägigen Monographien, z. B. von Kh. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, nachgelesen werden.

Die in Blondiermitteln enthaltenen Oxidationsmittel sind in der Lage, durch die oxidative Zerstörung des haareigenen Farbstoffes Melanin die Haarfaser aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein, für das Erzielen eines stärkeren Blondiereffektes wird üblicherweise eine Mischung aus Wasserstoffperoxid und Peroxodisulfatsalzen und/oder Peroxomonosulfatsalzen eingesetzt. Mit der Aufhellung geht jedoch auch eine Schädigung des Haares einher, da nicht nur die natürlichen farbgebenden Komponenten des Haares, sondern auch die übrigen Strukturbestandteile des Haares oxidativ geschädigt werden. Je nach Ausprägung des Schädigungsgrades reicht dieser von rauem, sprödem und schwieriger auskämmbarem Haar über eine verminderte Widerstandsfähigkeit und Reißfestigkeit des Haares bis hin zu Haarbruch. Je größer die Menge des eingesetzten Wasserstoffperoxids und gegebenenfalls der Peroxodisulfate ist, desto stärkere Schädigungen werden in der Regel auf der Keratinfaser hervorgerufen. Auch wenn die bislang auf dem Markt befindlichen Blondiermittel in der Regel gute Aufhellleistungen zeigen, so können sie aufgrund von Haarschädigung, langen Anwendungszeiten und der aufgrund der hohen Konzentrationen an Oxidations- und Alkalisierungsmittel möglichen Hautreizungen nicht als optimal angesehen werden. Es besteht daher weiterhin Bedarf nach Aufhellmitteln, welche eine gute Aufhellleistung zeigen, ohne gleichzeitig die Haarfaser zu schädigen.

Der Einsatz von kationischen Acylpyridiniumderivaten in Aufhellmitteln in Kombination mit bestimmten Coaktivatoren ist aus der WO2009/135700 A1 bekannt. DE 102007047685A1 lehrt, dass die Aufhellverstärkung eines kationischen Acylpyridiniumderivats der Formel (I) durch bestimmte Imidazol-Verbindungen als Co-Bleichaktivator verstärkt werden kann. Diesen Dokumenten ist jedoch kein Hinweis zu entnehmen, dass die Aufhellleistung solcher Acylpyridiniumderivate durch die Zugabe von Paraffinöl signifikant gesteigert werden kann. WO 2005/055966A1 lehrt, die Haltbarkeit einer künstlichen Haarfärbung durch Paraffinöl zu verbessern, da durch Paraffinöl die Farbstoffmoleküle besser in das Haar penetrieren.

Es ist die Aufgabe dieser Erfindung, die oben beschriebenen Nachteile handelsüblicher Blondiermittel zu verbessern. Insbesondere ist es Aufgabe der vorliegenden Erfindung, neuartige Mittel zum Aufhellen bzw. Blondieren von Haaren bereitzustellen, welche in ihrer Aufhellleistung den üblichen auf dem Markt befindlichen Mitteln vergleichbar oder überlegen sind, gleichzeitig jedoch eine verringerte Haarschädigung aufweisen.

In überraschender Weise konnte nun gefunden werden, dass der Einsatz einer Kombination von kationischen Acylpyridiniumverbindungen der allgemeinen Struktur (I), mindestens 5 Gew.-%, bezogen auf das Gesamtgewicht des Mittels, Paraffinöl und Wasserstoffperoxid die Haare viel stärker aufhellt, als es durch den Einsatz einer vergleichbaren Menge Wasserstoffperoxid oder in Kombination mit der Acylpyridiniumverbindung oder Paraffinöl allein möglich wäre. Bedingt durch die verbesserte Blondierleistung bei Anwendung des erfindungsgemäßen Mittels kann die Menge an eingesetztem Oxidationsmittel vermindert und die Haarschädigung hierdurch minimiert werden. Auch eine Verkürzung der Einwirkzeit unter Erzielung eines dem Stand der Technik entsprechenden Aufhelleffekts ist auf diese Weise möglich.

Die erfindungsgemäßen Mittel entfärben den natürlichen Farbstoff Melanin durch Oxidation, aber auch zuvor auf beziehungsweise in der keratinhaltigen Faser befindliche synthetische Farbstoffe können mit Hilfe der erfindungsgemäßen Mittel ausgeblichen werden.

Ein erster Gegenstand der Erfindung ist daher ein Mittel zum Aufhellen von keratinischen Fasern, dadurch gekennzeichnet, dass es in einem kosmetischen Träger
(i) mindestens ein kationisches Acylpyridiniumderivat der Formel (I), 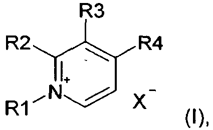 worin
   - R1: für eine C₁-C₆-Alkylgruppe, eine C₂-C₆-Alkenylgruppe, eine C₂-C₆-Hydroxy-alkylgruppe, eine C₁-C₆-Alkoxy-C₂-C₆-alkylgruppe, eine Carboxy-C₂-C₆-alkylgruppe, eine Aryl-C₁-C₆-alkylgruppe, eine Heteroaryl-C₁-C₆-alkylgruppe, eine Arylgruppe oder eine Heteroarylgruppe steht,
   - R2, R3 und R4: jeweils unabhängig voneinander für Wasserstoff, eine C₁-C₆-Alkylgruppe, ein Halogenatom oder eine C₁-C₆-Acylgruppe steht, mit der Massgabe, dass zumindest einer der Reste R2, R3 und R4 für eine C₁-C₆-Acylgruppe steht,
   - X⁻: für ein physiologisch verträgliches Anion steht,
(ii) mindestens 5 Gew.-%, bezogen auf das Gesamtgewicht des Mittels, Paraffinöl
   und
(iii) als Oxidationsmittel mindestens Wasserstoffperoxid enthält.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Aufhellen von Keratinfasern geeignet sind, steht prinzipiell einer Verwendung auch auf anderen Gebieten nichts entgegen.

Die erfindungsgemäßen Mittel enthaltend die Wirkstoffe in einem kosmetischen Träger. Der kosmetische Träger ist bevorzugt wässrig, alkoholisch oder wässrig-alkoholisch. Zum Zwecke der Haarbleiche sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Ein wässriger Träger enthält im Sinne der Erfindung mindestens 40 Gew.-%, insbesondere mindestens 50 Gew.-% Wasser. Unter wässrigalkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether enthalten. Bevorzugt sind dabei alle wasserlöslichen organischen Lösungsmittel.

Als ersten wesentlichen Inhaltstoff enthalten die erfindungsgemäßen Mittel mindestens ein Acylpyridiniumderivat gemäß Formel (I). Beispiele für die als Substituenten der Verbindungen der Formel (I) genannten werden nachfolgend, aber nicht beschränkend genannt: Beispiele für C₁-C₆-Alkylreste sind die Gruppen -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃. Beispiele für eine C₂-C₆-Alkenylgruppe sind eine Prop-2-enylgruppe (Allylgruppe), eine 2-Methyl-prop-2-enylgruppe, eine But-3-enylgruppe, eine But-2-enylgruppe, eine Pent-4-enylgruppe oder eine Pent-3-enylgruppe, wobei die Prop-2-enyl-gruppe bevorzugt ist. Beispiele für eine C₂-C₆-Hydroxyalkylgruppe sind -CH₂CH₂OH, -CH₂CH₂CH₂OH, -CH₂CH(OH)CH₃ und -CH₂CH₂CH₂CH₂OH, wobei die Gruppe -CH₂CH₂OH bevorzugt ist. Beispiele für C₁-C₆-Alkoxy-C₂-C₆-alkylgruppen sind die Gruppen -CH₂CH₂OCH₃, -CH₂CH₂CH₂OCH₃, -CH₂CH₂OCH₂CH₃, -CH₂CH₂CH₂OCH₂CH₃, -CH₂CH₂OCH(CH₃)₂, -CH₂CH₂CH₂OCH(CH₃)₂. Beispiele für eine Carboxy-C₁-C₆-alkylgruppe sind die Carboxymethylgruppe, die 2-Carboxyethylgruppe oder die 3-Carboxypropylgruppe. Beispiele für Aryl-C₁-C₆-alkylgruppen sind die Benzylgruppe und die 2-Phenylethylgruppe. Beispiele für eine Heteroaryl-C₁-C₆-alkylgruppe sind die Pyridin-2-ylmethylgruppe, die Pyridin-3-ylmethylgruppe, die Pyridin-4-ylmethylgruppe, die Pyrimidin-2-ylmethylgruppe, die Pyrrol-1-ylmethylgruppe, die Pyrrol-1-ylethyl-gruppe, die Pyrazol-1-ylmethylgruppe oder die Pyrazol-1-ylethylgruppe. Beispiele für eine Arylgruppe sind die Phenylgruppe, die 1-Naphthylgruppe oder die 2-Naphthylgruppe. Beispiele für eine Heteroarylgruppe sind die Pyridin-2-ylgruppe, die Pyridin-3-ylgruppe, die Pyridin-4-ylgruppe, die Pyrimidin-2-ylgruppe, die Pyrrol-1-ylgruppe, die Pyrrol-2-ylgruppe, die Pyrazol-1-ylgruppe, die Pyrazol-3-ylgruppe oder die Pyrazol-4-ylgruppe. Beispiele einer C₁-C₆-Acylgruppe sind Acetyl (1-Oxo-ethyl), 1-Oxo-propyl, 1-Oxo-butyl, 1-Oxo-Pentyl, 1-Oxo-2,2-dimethylpropyl, und 1-Oxo-hexyl.

In einer Ausführungsform der vorliegenden Erfindung sind solche Verbindungen gemäß Formel (I) bevorzugt, bei welchen der Rest R1 der allgemeinen Struktur (I) für eine C₁-C₆-Alkylgruppe, für eine C₂-C₆-Alkenylgruppe oder für eine C₂-C₆-Hydroxyalkylgruppe steht. Es ist erfindungsgemäß bevorzugt, wenn der Rest R1 für eine C₁-C₆-Alkylgruppe, bevorzugt für Methyl, Ethyl, n-Propyl oder Isopropyl und insbesondere bevorzugt für Methyl, steht.

Es hat sich herausgestellt, dass die Acylpyridiniumderivate gemäß Formel (I) erfindungsgemäß besonders vorteilhafte Eigenschaften besitzen, wenn sie die Acyl-Gruppe entweder in 2- oder 4-Position am Pyridin-Ring tragen. Bevorzugte Verbindungen der Formel (I) sind weiterhin solche Verbindungen, bei denen entweder der Rest R2 oder der Rest R4 für eine C₁-C₆-Acylgruppe, bevorzugt für eine Acetylgruppe, steht. Es ist weiterhin bevorzugt, wenn einer der Reste R2 oder R4 für eine Acetylgruppe steht, während der andere dieser Reste sowie der Rest R3 jeweils für Wasserstoff stehen. Eine weitere Ausführungsform der vorliegenden Erfindung ist daher dadurch gekennzeichnet, dass das Mittel als Acylpyridiniumderivat gemäß Formel (I) mindestens ein 2-Acetylpyridiniumderivat und/oder 4-Acetylpyridiniumderivat enthält. Geeignete Acetylpyridiniumderivate sind dabei insbesondere die physiologisch verträglichen Salze, die als Kation ein Acetylpyridiniumderivat, ausgewählt aus 4-Acetyl-1-methylpyridinium, 4-Acetyl-1-allylpyridinium, 4-Acetyl-1-(2-hydroxyethyl)pyridinium, 2-Acetyl-1-methylpyridinium, 2-Acetyl-1-allylpyridinium und 2-Acetyl-1-(2-hydroxyethyl)pyridinium, enthalten.

Es ist bevorzugt, wenn das Anion X⁻ gemäß Formel (I) ausgewählt wird aus Halogenid, insbesondere Chlorid, Bromid und Iodid, Benzolsulfonat, p-Toluolsulfonat, C₁-C₄-Alkylsulfonat, Trifluor-methansulfonat, Acetat, Triflluoracetat, Perchlorat, ½ Sulfat, Hydrogensulfat, Tetrafluorborat, Hexafluorphosphat oder Tetrachlorzinkat. Erfindungsgemäß vorteilhaft ist es, wenn das physiologisch verträgliche Anion X⁻ für ein Halogenidion (insbesondere Chlorid oder Bromid), Hydrogensulfat, p-Toluolsulfonat, Benzolsulfonat oder Acetat steht.

Insbesondere sind solche Mittel erfindungsgemäß bevorzugt, die dadurch gekennzeichnet sind, dass das Acylpyridiniumderivat gemäß Formel (I) ausgewählt ist aus der Gruppe, die gebildet wird aus 4-Acetyl-1-methylpyridinium-p-toluolsulfonat, 4-Acetyl-1-methylpyridinium-benzolsulfonat, 4-Acetyl-1-methylpyridiniumhydrogensulfat, 4-Acetyl-1-methylpyridiniumacetat, 4-Acetyl-1-allylpyridinium-p-toluolsulfonat, 4-Acetyl-1-allylpyridinium-benzolsulfonat, 4-Acetyl-1-allylpyridinium-hydrogensulfat, 4-Acetyl-1-allylpyridiniumacetat, 2-Acetyl-1-methylpyridinium-p-toluolsulfonat, 2-Acetyl-1-methylpyridinium-benzolsulfonat, 2-Acetyl-1-methylpyridiniumhydrogensulfat, 2-Acetyl-1-methylpyridiniumacetat, 2-Acetyl-1-allylpyridinium-p-toluolsulfonat, 2-Acetyl-1-allylpyridinium-benzolsulfonat, 2-Acetyl-1-allylpyridiniumhydrogensulfat und 2-Acetyl-1-allylpyridiniumacetat. Erfindungsgemäß bevorzugte Mittel sind dadurch gekennzeichnet, dass sie als das Acylpyridiniumderivat gemäß Formel (I) eine Verbindung, ausgewählt aus 4-Acetyl-1-methylpyridinium-p-toluolsulfonat und/oder 2-Acetyl-1-methylpyridinium-p-toluolsulfonat, insbesondere 4-Acetyl-1-methylpyridinium-p-toluolsulfonat, enthalten.

Eine Ausführungsform der vorliegenden Erfindung ist dabei dadurch gekennzeichnet, dass im erfindungsgemäßen Mittel die Acylpyridiniumderivate der Formel (I) in einer Menge von 0,1 bis 10 Gew.-%, insbesondere von 0,2 bis 4 Gew.-%, jeweils bezogen das Gesamtgewicht des Mittels, enthalten sind.

Als zweiten wesentlichen Inhaltstoff enthalten die erfindungsgemäßen Mittel weiterhin mindestens 5 Gew.-%, bezogen auf das Gesamtgewicht des Mittels, Paraffinöl.

Es hat sich herausgestellt, dass sich die Aufhellleistung der Mittel insbesondere dann synergistisch steigern lässt, wenn das Mittel einen höheren Anteil, wenigstens von 5 Gew.-%, besser von 10 Gew.-%, an Paraffinöl enthält.

Eine Ausführungsform der vorliegenden Erfindung ist daher ein Mittel, welches dadurch gekennzeichnet ist, dass es mindestens 5 Gew.-%, bevorzugt mindestens 10 Gew.-% und besonders bevorzugt von 10 bis 40 Gew.-%, insbesondere von 15 bis 30 Gew.-%, jeweils bezogen auf das Gesamtgewicht des Mittels, an Paraffinöl enthält.

Als dritten wesentlichen Bestandteil enthält das erfindungsgemäße Mittel als Oxidationsmittel mindestens Wasserstoffperoxid. Bevorzugt wird Wasserstoffperoxid selbst als wässrige Lösung verwendet. Wasserstoffperoxid kann aber auch in Form einer festen Anlagerungsverbindung von Wasserstoffperoxid an anorganische oder organische Verbindungen, wie Natriumpercarbamid, Polyvinylpyrrolidinon H₂O₂ (n ist eine positive ganze Zahl größer 0), Harnstoffperoxid und Melaminperoxid, eingesetzt werden.

Wasserstoffperoxid ist im anwendungsbereiten Mittel bevorzugt zu 0,1 bis 25 Gew.-%, insbesondere bevorzugt zu 1 bis 20 Gew.-% und besonders bevorzugt zu 4,5 bis 9 Gew.-%, jeweils berechnet auf 100%iges Wasserstoffperoxid und bezogen auf das Gesamtgewicht des anwendungsbereiten Mittels, enthalten.

Unter Berücksichtigung der bisher genannten bevorzugten Ausführungsformen stellt es eine ganz spezielle und ausdrücklich bevorzugte Ausführungsform dar, wenn das Mittel zum Aufhellen von keratinischen Fasern in einem kosmetischen Träger als erste Komponente mindestens eine Verbindung, ausgewählt aus der Gruppe, die gebildet wird aus 4-Acetyl-1-methylpyridinium-p-toluolsulfonat, 4-Acetyl-1-methylpyridinium-benzolsulfonat, 4-Acetyl-1-methylpyridiniumhydrogensulfat, 4-Acetyl-1-methylpyridiniumacetat, 4-Acetyl-1-allylpyridinium-p-toluolsulfonat, 4-Acetyl-1-allyl-pyridinium-benzolsulfonat, 4-Acetyl-1-allylpyridiniumhydrogensulfat, 4-Acetyl-1-allylpyridinium-acetat, 2-Acetyl-1-methylpyridinium-p-toluolsulfonat, 2-Acetyl-1-methylpyridinium-benzolsulfonat, 2-Acetyl-1-methylpyridiniumhydrogensulfat, 2-Acetyl-1-methylpyridiniumacetat, 2-Acetyl-1-allyl-pyridinium-p-toluolsulfonat, 2-Acetyl-1-allylpyridinium-benzolsulfonat, 2-Acetyl-1-allylpyridinium-hydrogensulfat und 2-Acetyl-1-allylpyridiniumacetat, enthält, als zweite Komponente mindestens 10 Gew.-% Paraffinöl (Paraffinum liquidum) enthält, und als dritte Komponente Wasserstoffperoxid enthält.

Besonders bevorzugt sind schließlich Mittel, die 0,2 bis 4,0 Gew.-% 4-Acetyl-1-methylpyridinium-p-toluolsulfonat, 10 bis 40 Gew.-% Paraffinöl (Paraffinum liquidum) und 2,0 bis 12,0 Gew.-% Wasserstoffperoxid enthalten, oder Mittel, die 0,2 bis 4,0 Gew.-% 2-Acetyl-1-methylpyridinium-p-toluolsulfonat, 10 bis 40 Gew.-% Paraffinöl (Paraffinum liquidum) und 2,0 bis 12,0 Gew.-% Wasserstoffperoxid enthalten, worin sich die Gewichtsangaben jeweils auf das Gesamtgewicht des Mittels beziehen.

Erfindungsgemäß kann das Mittel zusammen mit einem Katalysator auf das Haar aufgebracht werden, der die Oxidation der Farbstoffvorprodukte zusätzlich aktiviert. Solche Katalysatoren sind z. B. bestimmte Enzyme, Iodide, Chinone oder Metallionen. Hierfür geeignete Enzyme sind z. B. Peroxidasen, die die Wirkung geringer Mengen an Wasserstoffperoxid deutlich verstärken können. Ein Einsatz bestimmter Metallionen oder -komplexe kann ebenfalls bevorzugt sein. Besonders geeignet sind dabei Zn²⁺, Cu²⁺ und Mn²⁺.

Weiterhin hat es sich als vorteilhaft erweisen, wenn die Mittel mindestens einen Stabilisator oder Komplexbildner enthalten. Besonders bevorzugte Stabilisatoren sind Phenacetin, Alkalibenzoate (Natriumbenzoat) und Salicylsäure.

Erfindungsgemäß bevorzugt ist auch der Einsatz von sogenannten Komplexbildnern. Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mindestens "zweizähnig" ist. Gebräuchliche und im Rahmen der vorliegenden Erfindung bevorzugte Chelatkomplexbildner sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Auch komplexbildende Polymere, also Polymere, die entweder in der Hauptkette selbst oder seitenständig zu dieser funktionelle Gruppen tragen, die als Liganden wirken können und mit geeigneten Metall-atomen in der Regel unter Bildung von Chelat-Komplexen reagieren, sind erfindungsgemäß einsetzbar. Die Polymer-gebundenen Liganden der entstehenden Metall-Komplexe können dabei aus nur einem Makromolekül stammen oder aber zu verschiedenen Polymerketten gehören. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/ oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Blondierprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Bei den pH-Werten im Sinne der vorliegenden Erfindung handelt es sich um pH-Werte, die bei einer Temperatur von 22 °C gemessen wurden.

Andererseits kann es aus Stabilitätsgründen sinnvoll sein, bestimmte Inhaltstoffe bei einem neutralen oder schwach sauren pH-Wert zu lagern und erst zum Zeitpunkt der Anwendung einem alkalischen Milieu auszusetzen. Zu diesen Inhaltstoffen gehört Wasserstoffperoxid, aber auch die kationischen Acylpyridiniumderivate der Formel (I) werden bevorzugt bei neutralen oder schwach sauren pH-Wert gelagert.

Es kann daher bevorzugt sein, wenn das erfindungsgemäße Mittel einen schwach sauren pH-Wert besitzt. Eine weitere Ausführungsform der vorliegenden Erfindung ist daher dadurch gekennzeichnet, dass das Mittel als Mittel M1 einen pH-Wert von pH 2 bis pH 6, bevorzugt vom pH 2,5 bis pH 4,5 besitzt.

Zur Einstellung des pH-Werts sind dem Fachmann gängige Acidifizierungs- und Alkalisierungsmittel geläufig. Die zur Einstellung des pH-Wertes verwendbaren Alkalisierungsmittel werden typischerweise gewählt aus Ammoniak, anorganischen Salzen, insbesondere der Alkali- und Erdalkalimetalle, organischen Alkalisierungsmitteln, insbesondere Alkanolaminen, Aminen und basische Aminosäuren. Erfindungsgemäß bevorzugte Acidifizierungsmittel sind Genuss-Säuren, wie Milchsäure, Zitronensäure, Essigsäure, Äpfelsäure oder Weinsäure, verdünnte Mineralsäuren und deren in Wasser sauer reagierenden Salze sowie organische Phosphon- oder Sulfonsäuren.

Um die Aufhellleistung zu verbessern, besitzen die anwendungsbereiten Blondiermittel jedoch bevorzugt einen alkalischen pH-Wert. Daher ist es bevorzugt, ein Mittel des ersten Erfindungsgegenstands unmittelbar vor der Anwendung auf den keratinischen Fasern auf einen alkalischen pH-Wert zwischen 6 und 12, insbesondere zwischen 9 und 11, einzustellen.

Ein weiterer Gegenstand der vorliegenden Erfindung ist daher ein anwendungsbereites Mittel zum Aufhellen von keratinischen Fasern, insbesondere menschlichen Haaren, welches dadurch gekennzeichnet, dass es unmittelbar vor Anwendung durch Vermischen eines Mittels M1 gemäß dem ersten Erfindungsgegenstand und eines Mittels M2, enthaltend in einem kosmetisch verträglichen Träger mindestens ein Alkalisierungsmittel, hergestellt wird und dass das anwendungsbereite Mittel einen pH-Wert von pH 6 bis pH12, bevorzugt pH 9 bis pH 11 besitzt.

Die erfindungsgemäß verwendbaren Alkalisierungsmittel des Mittels M2 werden bevorzugt aus der Gruppe, die gebildet wird aus Ammoniak, anorganischen Alkalisierungsmitteln, insbesondere der Alkali- und Erdalkalimetalle, organischen Alkalisierungsmitteln, insbesondere Alkanolaminen, Aminen und basische Aminosäuren.

Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat. Besonders bevorzugt sind Natriumhydroxid und/oder Kaliumhydroxid. D

Die als erfindungsgemäßes Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Amino-pentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß besonders bevorzugte Alkanolamine werden ausgewählt aus der Gruppe 2-Aminoethan-1-ol, 2-Amino-2-methylpropan-1-ol und 2-Amino-2-methyl-propan-1,3-diol. Die basischen Aminosäuren werden bevorzugt ausgewählt aus der Gruppe, die gebildet wird aus L-Arginin, D-Arginin, D/L-Arginin, L-Lysin, D-Lysin, D/L-Lysin, besonders bevorzugt L-Arginin, D-Arginin und D/L-Arginin.

Besonders bevorzugt als Alkalisierungsmittel enthält das Mittel M2 jedoch Ammoniak. Eine Ausführungsform der vorliegenden Erfindung ist daher dadurch gekennzeichnet, dass das Mittel M2 als Alkalisierungsmittel Ammoniak enthält.

Erfindungsgemäße, anwendungsbereite Mittel sind vorzugsweise wässrige, fließfähige Zubereitungen. Die erfindungsgemäßen Mittel können weiterhin alle für solche Zubereitungen bekannten Wirk-, Zusatz- und Hiltfsstoffe enthalten. Die anwendungsbereiten Mittel als Mischung aus Mittel M1 und M2 können dabei oberflächenaktive Substanzen, ausgewählt aus anionischen sowie nichtionischen, zwitterionischen, amphoteren und kationischen Tensiden enthalten.

Anionische Tenside sind gekennzeichnet durch eine wasserlöslich machende, anionische Gruppe wie eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für solche anionischen Tenside sind, jeweils in Form der Natrium-, Kalium- und Ammonium- sowie der Mono, Di- und Trialkanolammoniumsalze mit 2 bis 4 C-Atomen in der Alkanolgruppe, lineare und verzweigte Fettsäuren mit 8 bis 30 C-Atomen (Seifen); Ethercarbonsäuren, insbesondere der Formel RO(CH₂CH₂O)ₓCH₂COOH, in der R eine lineare Alkylgruppe mit 8 bis 30 C-Atomen und x = 0 oder 1 bis 16 ist; Acylsarcoside; Acyltauride; Acylisethionate; Sulfobernsteinsäuremono- und -dialkylester sowie Sulfobernsteinsäuremono-alkylpolyoxyethylester; lineare Alkansulfonate; lineare α-Olefinsulfonate; Sulfonate ungesättigter Fettsäuren; α-Sulfofettsäuremethylester von Fettsäuren; Alkylsulfate und Alkylethersulfate, insbesondere der Formel RO(CH₂CH₂O)ₓSO₃H, in der R für eine lineare Alkylgruppe mit 8 bis 30 C-Atomen und x für 0 oder eine Zahl von 1 bis 12 steht; Gemische oberflächenaktiver Hydroxysulfonate; sulfatierte Hydroxyalkylpolyethylen- und/oder Hydroxyalkylenpropylenglykolether; Ester der Weinsäure und Zitronensäure mit Alkoholen; Alkyl- und/oder Alkenyletherphosphate der Formel RO(C₂H₄O)ₓP(=O)(OH)(OR'), worin R für einen aliphatischen, gegebenenfalls ungesättigten Kohlenwasserstoffrest mit 8 bis 30 Kohlenstoffatomen, R' für Wasserstoff, einen Rest (CH₂CH₂O)_{y}R und x und y unabhängig voneinander für eine Zahl von 1 bis 10 steht; sulfatierte Fettsäurealkylenglykolester der Formel RC(O)O(alkO)ₙSO₃H, in der R für einen linearen oder verzweigten, aliphatischen, gesättigten und/oder ungesättigten Alkylrest mit 6 bis 22 C-Atomen, alk für CH₂CH₂, CHCH₃CH₂ und/oder CH₂CHCH₃ und n für eine Zahl von 0,5 bis 5 steht; sowie Monoglyceridsulfate und Monoglyceridethersulfate.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Beispiele solcher zwitterionischen Tenside sind die Betaine wie die N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline mit jeweils 8 bis 18 C-Atomen in der Alkyl- oder Acylgruppe sowie Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Übüche amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Beispielhafte amphotere Tenside sind N-Kokosalkylaminopropionat, Kokosacylaminoethylaminopropionat und C₁₂-C₁₈-Acylsarcosin.

Nichtionische Tenside und Emulgatoren enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise Anlagerungsprodukte von 1 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe; mit einem Methyl- oder C₂-C₆-Alkylrest endgruppenverschlossene Anlagerungsprodukte von 1 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe; Polyglycerinester und alkoxylierte Polyglycerinester; Polyolfettsäureester; höher alkoxylierte, propoxylierte und insbesondere ethoxylierte, Mono-, Di-und Triglyceride mit Alkoxylierungsgrad von größer als 5, wie beispielsweise Glycerinmonolaurat + 20 Ethylenoxid und Glycerinmonostearat + 20 Ethylenoxid; Aminoxide; Hydroxymischether; Sorbitanfettsäureester und Anlagerungeprodukte von Ethylenoxid an Sorbitanfettsäureester wie beispielsweise die Polysorbate und Sorbitanmonolaurat + 20 Mol Ethylenoxid (EO); Zuckerfettsäureester und Anlagerungsprodukte von Ethylenoxid an Zuckerfettsäureester; Anlagerungsprodukte von Ethylenoxid an Fettsäurealkanolamide und Fettamine; Fettsäure-N-alkylglucamide; Alkylphenole und Alkylphenolalkoxylate mit 6 bis 21, insbesondere 6 bis 15 Kohlenstoffatomen in der Alkylkette und 5 bis 30 Ethylenoxid- und/oder Propylenoxid-Einheiten; Alkylpolyglykoside entsprechend der allgemeinen Formel RO-(Z)ₓ, wobei R für Alkyl, Z für Zucker sowie x für die Anzahl der Zuckereinheiten steht.

Zu den nichtionischen Emulgatoren im Sinne der Erfindung zählen weiterhin die Polymerisationsprodukte aus Ethylenoxid und Propylenoxid an gesättigte oder ungesättigte Fettalkohole; Fettsäureester mehrwertiger Alkohole mit gesättigten oder ungesättigten Fettsäuren; Alkylester von gesättigten oder ungesättigten Fettsäuren oder Alkylphenole und deren Alkoxylate; insbesondere Ethylenglykolether von Fettalkoholen; gemischte Ethylen- und Propylenglykolether mit Fettalkoholen; Fettsäureester an Sorbitan sowie Polyethylenglykol; Ester von nicht hydroxylierten C₆-C₃₀-Alkylmonocarbonäuren mit Polyethylenglykol; und Anlagerungsprodukte von Alkylphenolen an Ethylen- und/oder Propylenoxid.

Erfindungsgemäß bevorzugt sind in anwendungsbereiten Mitteln kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammonium-chloride sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Alkylamidoamine werden üblicherweise durch Amidierung natürlicher oder synthetischer Fettsäuren und Fettsäureschnitte mit Dialkylaminoaminen hergestellt, wie Stearamidopropyl-dimethylamin. Ebenfalls bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex, Dehyquart und Armocare vertrieben. Die Produkte Armocare VGH-70, ein N,N-Bis(2-Palmitoyloxyethyl)di-methylammoniumchlorid, sowie Dehyquart F-75, Dehyquart C-4046, Dehyquart L80 und Dehyquart AU-35 sind Beispiele für solche Esterquats. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer bevorzugten Ausführungsform können anionische, nicht-ionische, zwitterionische und/oder amphotere Tenside sowie deren Mischungen bevorzugt sein.

Die Mittel M1 und/oder M2 können weitere Wirk-, Hilfs- und Zusatzstoffe enthalten. Weitere, erfindungsgemäß einsetzbare Wirk-, Hilfs- und Zusatzstoffe sind beispielsweise anionische Polymere (wie Carbomere, Co- und Crosspolymere von Acrylsäure, Methacrylsäure, Maleinsäure, Itaconsäure und gegebenenfalls weiteren nichtionischen Monomeren); nichtionische Polymere (wie Vinylpyrrolidinon/Vinylacrylat-Copolymere, Polyvinylpyrrolidinon und Vinylpyrrolidinon/VinylacetatCopolymere und Polysiloxane); zwitterionische und amphotere Polymere (wie Acrylamidopropyltri-methylammoniumchlorid/Acrylat-Copolymere und Octylacrylamid/Methyl-methacrylat/tert-Butylaminoethylmethacrylat/2-Hydroxypropylmethacrylat-Copolymere); Verdickungsmittel (wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie Bentonit oder vollsynthetische Hydrokolloide wie z.B. Polyvinylalkohol); Strukturanden (wie Zucker, Maleinsäure und Milchsäure) und Konsistenzgeber (wie Zuckerester, Polyolester oder Polyolalkylether); Proteinhydrolysate (insbesondere Elastin-, Kollagen-, Keratin-, Milcheiweiß-, Sojaprotein- und Weizenproteinhydrolysate, deren Kondensationsprodukte mit Fettsäuren); Parfümöle; Pflegeöle; Cyclodextrine; Entschäumer wie Silicone; Farbstoffe und Pigmente zum Anfärben des Mittels; Antischuppenwirkstoffe (wie Piroctone Olamine, Zink Omadine und Climbazol); Lichtschutzmittel (insbesondere derivatisierte Benzophenone, Zimtsäure-Derivate und Triazine); Wirkstoffe (wie Allantoin, Pyrrolidoncarbonsäuren, Cholesterin und deren Salze); weitere Fette und Wachse (wie Fettalkohole, Bienenwachs, Montanwachs und Paraffine); Quell- und Penetrationsstoffe (wie Glycerin, Propylenglykolmonoethytether, Carbonate, Hydrogencarbonate, Guanidine, Harnstoffe sowie primäre, sekundäre und tertiäre Phosphate); Trübungsmittel (wie Latex, Styrol/PVP- und Styrol/Acrylamid-Copolymere); Perlglanzmittel (wie Ethylenglykolmono- und -distearat sowie PEG-3-distearat); Treibmittel (wie Propan-Butan-Gemische, N₂O, Dimethylether, CO₂ und Luft) sowie Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Mittel nicht nur als reine Aufhellmittel, d.h. als sogenannte Blondiermittel, sondern auch als mattierende Aufhellmittel bereitgestellt werden, die gleichzeitig mit der Aufhellung auch eine Mattierung der Keratinfasern bewirken, so dass die bei Blondierungen häufig auftretenden, aber unerwünschten Farbverschiebungen in rötliche oder orange Bereiche durch eine geringe Färbung, insbesondere in kühlen Farbtönen, ausgeglichen wird.

Für solche Mattierungen werden als färbende Komponente sogenannte direktziehende Farbstoffe ("Direktzieher") eingesetzt. Hierbei handelt es sich um Farbstoffmoleküle, die direkt auf das Substrat aufziehen und keinen oxidativen Prozess zur Ausbildung der Farbe benötigen.

In einer Ausführungsform der vorliegenden Erfindung können die erfindungsgemäßen, anwendungsbereiten Mittel mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Die direktziehenden Farbstoffe werden jeweils bevorzugt in einer Menge von 0,0001 bis 5,0 Gew.-%, bevorzugt von 0,001 bis 1,5 Gew.-%, jeweils bezogen auf die gesamte Anwendungszubereitung, eingesetzt. Die Gesamtmenge an direktziehenden Farbstoffen beträgt vorzugsweise höchstens 1,0 Gew.-%.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen. Bevorzugte kationische direktziehende Farbstoffe sind kationische Triphenylmethanfarbstoffe, wie Basic Blue 7, Basic Blue 26, Basic Violet 2 und Basic Violet 14, aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Kationische direktziehende Farbstoffe, die unter dem Warenzeichen Arianor^{®} vertrieben werden, sind erfindungsgemäß ebenfalls besonders bevorzugte kationische direktziehende Farbstoffe. Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol. Erfindungsgemäß bevorzugte Farbstoffkombinationen sind solche, die mindestens die Kombination aus Tetrabromphenolblau und Acid Red 92; Tetrabromphenolblau und Acid Red 98; Tetrabromphenolblau und Acid Red 94; Tetrabromphenolblau und Acid Red 87 oder Tetrabromphenolblau und Acid Red 51.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend. In diesen Fällen wird in handelsüblichen Blondiermittel in der Regel eine Kombination aus Wasserstoffperoxid und Persulfaten bzw. Peroxodisulfaten eingesetzt. Es hat sich gezeigt, dass die Zugabe der erfindungsgemäßen Acylpyridiniumderivate der allgemeinen Struktur (I) und mindestens 5 Gew.-%, bezogen auf das Gesamtgewicht des Mittels, Paraffinöl bei Wasserstoffperoxid allein bereits zu einer deutlichen Verbesserung der Aufhellleistung führt, so dass in der Regel auf die Zugabe von Persulfaten verzichtet werden kann.

Es kann jedoch, sollte der Verbraucher den Wunsch nach einer sehr starken Blondierung verspüren, in einer weiteren Ausführungsform bevorzugt sein, wenn neben der kationischen Acylpyridiniumverbindung der allgemeinen Struktur (I), mindestens 5 Gew.-%, bezogen auf das Gesamtgewicht des Mittels, Paraffinöl und Wasserstoffperoxid zusätzlich mindestens ein anorganisches Persulfatsalz bzw. Peroxodisulfatsalz in dem Mittel zum Aufhellen der keratinischen Fasern enthalten ist.

Bevorzugte Peroxodisulfatsalze sind Ammoniumperoxodisulfat, Kaliumperoxodisulfat und Natriumperoxodisulfat. Die Peroxodisulfatsalze können in einer Menge von 0,1 bis 25 Gew.-%, insbesondere in einer Menge von 0,5 bis 15 Gew.-%, bezogen auf das Gesamtgewicht des anwendungsbereiten Mittels, enthalten sein. Diese Peroxodisulfate werden dem Mittel ebenfalls bevorzugt erst unmittelbar vor der Anwendung beigemischt, um Instabilitäten bei der Lagerung zu vermeiden.

Das erfindungsgemäße Mittel wird vor der Anwendung durch Vermischen der Alkalisierungszubereitung M2 und der Oxidationszubereitung M1 hergestellt. Es ist daher vorteilhaft, dem Anwender beide Zubereitungen in einem Set anzubieten. Daher ist eine bevorzugte Darreichungsform des anwendungsbereiten Mittels eine getrennte Verpackungseinheit, worin die Mittel M1 und M2 jeweils getrennt voneinander verpackt vorliegen.

Ein weiterer Gegenstand der vorliegenden Erfindung ist daher eine Mehrkomponentenverpackungseinheit (Kit-of-Parts), umfassend mindestens zwei getrennt voneinander konfektionierte Container, wobei ein erster Container C1 mindestens ein Mittel M1 gemäß dem ersten Erfindungsgegenstand und ein zweiter Container C2 mindestens ein Mittel M2, enthaltend in einem kosmetisch verträglichen Träger mindestens ein Alkalisierungsmittel, beinhaltet.

Unter Container wird im Rahmen der vorliegenden Erfindung eine Umhüllung verstanden, die in Form einer gegebenenfalls wieder-verschließbaren Flasche, einer Tube, einer Dose, eines Tütchens, eines Sachets oder ähnlichen Umhüllungen vorliegt. Dem Umhüllungsmaterial sind erfindungsgemäß keine Grenzen gesetzt. Bevorzugt handelt es sich jedoch dabei um Umhüllungen aus Glas oder Kunststoff.

Weiterhin kann es erfindungsgemäß besonders vorteilhaft sein, wenn das genannte Kit-of-Parts mindestens ein weiteres Haarbehandlungsmittel in einem getrennten Container enthält, insbesondere eine Konditioniermittelzubereitung oder ein Blondierpulver mit Peroxodisulfaten. Darüber hinaus kann die Verpackungseinheit Applikationshilfen, wie Kämme, Bürsten oder Pinsel, persönliche Schutzkleidung, insbesondere Ein-Weg-Handschuhe, sowie gegebenenfalls eine Gebrauchsanleitung umfassen.

Hinsichtlich der bevorzugten Ausführungsführungsformen der Mittel M1 und M2 gelten mutatis mutandis die obigen Ausführungen der vorangehenden Erfindungsgegenstände.

Ein weiterer Erfindungsgegenstand ist ein Verfahren zur Farbveränderung keratinischer Fasern, insbesondere menschlicher Haare, welches dadurch gekennzeichnet ist, dass aus einer Mehrkomponentenverpackungseinheit gemäß dem vorangegangenen Erfindungsgegenstand die beiden Mittel M1 und M2 in einem der Container C2 oder C1 vereinigt werden, der wiederverschlossene Container daraufhin geschüttelt wird, und das im Container resultierende, anwendungsbereite Farbveränderungsmittel anschließend auf die Fasern aufgebracht wird, für eine Einwirkdauer von 5 bis 60 min, bevorzugt 30 bis 45 min auf den Fasern belassen und schließlich ausgespült wird.

Die Anwendungstemperaturen können in einem Bereich zwischen 15 und 40 °C liegen. Nach der Einwirkungszeit wird das verbleibende Mittel durch Ausspülen von dem Haar entfernt. Das Nachwaschen mit einem Shampoo entfällt, wenn ein stark tensidhaltiger Träger verwendet wurde.

Im Rahmen dieses Gegenstandes der Erfindung gelten mutatis mutandis die oben getroffenen Aussagen analog.

Ein weiterer Gegenstand der vorliegenden Erfindung ist die kosmetische Verwendung eines Mittels, welches durch Mischung der beiden Mittel M1 und M2 hergestellt wurde, zum Aufhellen von keratinhaltigen Fasern, insbesondere menschlichen Haaren. Es hat sich dabei gezeigt, dass durch die Kombination eines kationischen Acylpyridiniumderivats der Formel (I) mit mindestens 5 Gew.-%, bezogen auf das Gesamtgewicht des Mittels, Paraffinöl die Aufhellleistung von Blondiermitteln signifikant steigern lässt. Eine synergistische Wirkung der Inhaltstoffe wird insbesondere erzielt, wenn Paraffinöl in größeren Gewichtsanteilen, zumindest 10 Gew.-% im Mittel vorliegt.

Ein weiterer Gegenstand der vorliegenden Erfindung ist ein Verfahren zur Herstellung eines anwendungsbereiten Mittels zum Aufhellen von keratinischen Fasern, insbesondere menschlichen Haaren, wonach unmittelbar vor Anwendung ein Mittel M1 gemäß einem der Ansprüche 1 bis 5 und ein Mittel M2, enthaltend in einem kosmetisch verträglichen Träger mindestens ein Alkalisierungsmittel, miteinander vermischt werden, wobei das anwendungsbereite Mittel einen pH-Wert von pH 6 bis pH 12, bevorzugt pH 9 bis pH 11 besitzt.

### Beispiele

### 1.1 Herstellung einer Blondiercreme (Tabelle 1)

| **Rohstoff** | **Gew.-%** |
|---|---|
| Ammoniumcarbomer, wässrig, 1,0 Gew.-% | 15,00 |
| Natrium Cetearylsulfat | 0,70 |
| Natrium Laurylethersulfat (2 EO), wässrig, Gew.-27% | 4,40 |
| Kalium Oleat, wässrig, 12,5 Gew.-% | 3,00 |
| Cetearyl Alcohol | 12,00 |
| Ceteareth-20 | 3,00 |
| 2-Octyldodecanol | 2,00 |
| Cutina AGS | 2,00 |
| Cutina GMS SE | 2,00 |
| Titandioxid | 0,50 |
| Phospholipid EFA | 0,10 |
| Tetranatrium EDTA | 0,20 |
| Merquat Plus 3330 | 0,50 |
| Ascorbinsäure | 0,05 |
| Puricare LS 9658 | 1,00 |
| Parfum | qs |
| Ammoniak, 25 Gew.-%, wässrig | 15,00 |
| Wasser | ad 100 |

| | |
|---|---|
| *eingesetzte Rohstoffe: Cutina AGS (INCI-Bezeichnung: Glycol Distearate (Cognis)); Cutina GMS SE (INCI-Bezeichnung: Glyceryl Stearate (Cognis)); Phospholipid EFA (ca. 30%, INCI-Bezeichnung: Linoleamidopropyl PG-dimonium chloride phosphate, Propylene glycol (Uniqema)); Merquat Plus 3330 (ca. 10% ; INCI-Bezeichnung: Polyquaternium-39 (Nalco)); Puricare LS 9658 (ca. 1%; INCI-Bezeichnung: Glycerin, Moringa Pterygosperma Seed Extrakt (Laboratoires Serobiologiques). | |

### 1.2 Oxidationsmittelzubereitungen (Tabelle 2)

| **Rohstoff** | **Oxidationsmittelzubereitung [Gew.%]** | | | |
|---|---|---|---|---|
| | **E1 (Vgl.)** | **E2 (Vgl.)** | **E3 (Vgl.)** | **E4 (Erf.)** |
| Natriumbenzoat | 0,04 | 0,04 | 0,04 | 0,04 |
| Dinatriumpyrophosphat | 0,10 | 0,10 | 0,10 | 0,10 |
| Etidronsäure, wässrig, 60 Gew.-% | 0,25 | 0,25 | 0,25 | 0,25 |
| Kaliumhvdroxid, wässrig, 50 Gew.-% | 0,12 | 0,12 | 0,12 | 0,12 |
| Cetearylalkohol | 4,00 | 4,00 | 4,00 | 4,00 |
| Ceteareth-20 | 1,00 | 1,00 | 1,00 | 1,00 |
| Bienenwachs | 0,30 | 0,30 | 0,30 | 0,30 |
| Paraffinum Liquidum | -- | 20,00 | -- | 20,00 |
| 4-Acetyl-1-methylpyridinium-p-toluolsulfonat | -- | -- | 2,00 | 2,00 |
| Wasserstoffperoxid, wässrig, 50 Gew.-% | 23,30 | 23,30 | 23,30 | 23,30 |
| Wasser | ad 100 | ad 100 | ad 100 | ad 100 |

Bei den Rezepturen E1 bis E3 handelt es sich um nicht erfindungsgemäße Vergleichsrezepturen, die Rezepturen E4 ist ein erfindungsgemäßes Beispiel mit dem Bleichaktivator 4-Acetyl-1-methyl-pyridinium-p-toluolsulfonat und Paraffinum Liquidum. Die anwendungsbereiten Mittel wurden durch Vermischen der Blondiercreme mit einer Oxidationsmittelzubereitung im gewünschten Verhältnis hergestellt (Tabellen 3 und 4).

Für den Blondierprozess wurde auf Strähnen dunkelblonden und braunen Haares (Codes: Kerling NH 6/0 bzw. Kerling NH 3/0) von ca. 0,7 g Gewicht die 4-fache Menge der fertigen Anwendungsmischung appliziert. Nachdem die Strähnen für 30 Minuten bei 32 °C blondiert wurden, wurden sie mit einem handelsüblichen Shampoo gewaschen und mit einem Föhn getrocknet.

### 2.1 Auswertung der Aufhellleistung

Jede Haarsträhne wurde vor und nach dem Bleichvorgang farbmetrisch vermessen. Als Maß für die Aufhellleistung der jeweiligen Rezeptur wurde der ΔL-Wert gemäß folgender Formel herangezogen: ΔL = L(nachher) - L(vorher) mit
L(nachher) = Helligkeit der Strähne nach dem Bleichen;
L(vorher) = Helligkeit der unbehandelten Strähne.

Für jede Rezeptur und jeden Haartyp erfolgte eine Zwölffachbestimmung, aus den Einzelwerten wurde jeweils der Mittelwert gebildet. Je größer der ΔL-Wert ist, desto besser ist die Aufhellleistung der jeweiligen Rezeptur.

### Aufhellleistung bei dunkelblonden Strähnen (Kerling 6/0)

| Mittel | Oxidationsmittel M1 | Blondiercreme M2 | Verhältnis M1 / M2 (Gewichtsteile) | L-Wert | Aufhellleistung [ΔL] |
|---|---|---|---|---|---|
| **0a** | unbehandelt | | | 27,1 | - |
| **1a** | E1 | BC | 1 : 1 | **42,0** | **14,9** |
| **2a** | E1 | BC | 1 : 2 | **44,4** | **17,3** |
| **3a** | E2 | BC | 1 : 2 | **46,0** | **18,9** |
| **4a** | E3 | BC | 1 : 2 | **46,4** | **19,3** |
| **5a** | E4 | BC | 1 : 2 | **51,1** | **24,0** |

### Aufhellleistung bei braunen Strähnen (Kerling 3/0)

| Mittel | Oxidationsmittel M1 | Blondiercreme M2 | Verhältnis M1 / M2 (Gewichtsteile) | L-Wert | Aufhellleistung [ΔL] |
|---|---|---|---|---|---|
| **0b** | unbehandelt | | | 19,1 | -- |
| **1b** | E1 | BC | 1 : 2 | 26,7 | 7,6 |
| **2b** | E1 | BC | 1 : 2 | **27,5** | **8,4** |
| **3b** | E2 | BC | 1 : 2 | **30,3** | **11,2** |
| **4b** | E3 | BC | 1 : 2 | **29,0** | **9,9** |
| **5b** | E4 | BC | 1 : 2 | **36,8** | **17,7** |

### 2.2 Deutung der Ergebnisse

Eine Abschätzung der Bleichwirkungen der unterschiedlichen Rezepturen lässt sich durch den Vergleich der ΔL-Werte treffen. Es ist eindeutig ersichtlich, dass mit der erfindungsgemäßen Kombination aus Paraffinöl und Acylpyridiniumderivat signifikant größere ΔL Werte - und damit eine bessere Aufhellung - erzielt werden konnten, als es ohne Zusatz oder durch den Einsatz von einer der beiden Komponenten möglich war.

Es wird weiterhin deutlich, dass die Verbesserung der Aufhellleistung deutlich über den simplen additiven Effekt der beiden Einzelkomponenten hinausgeht und damit ein synergistischer Effekt vorliegt, der nicht zu erwarten war:

| Zusätzlicher Aufhelleffekt [ΔΔL] auf Kerling 6/0 | |
|---|---|
| durch Zugabe von Paraffinöl: | + 1,6 (aus Mittel 2a und 3a) |
| durch Zugabe von Acylpyridiniumderivat: | + 2,0 (aus Mittel 2a und 4a) |
| durch Zugabe von Paraffinöl und Acylpyridiniumderivat | + **6,7** (aus Mittel 2a und 5a) |
| | |

| Zusätzlicher Aufhelleffekt [ΔΔL] auf Kerling 3/0 | |
|---|---|
| durch Zugabe von Paraffinöl: | + 2,8 (aus Mittel 2b und 3b) |
| durch Zugabe von Acylpyridiniumderivat: | + 1,5 (aus Mittel 2b und 4b) |
| durch Zugabe von Paraffinöl und Acylpyridiniumderivat | + **9,3** (aus Mittel 2b und 5b) |

Durch den Einsatz dieser speziellen Kombination konnte daher gegenüber dem existierenden Stand der Technik eine wesentliche Verbesserung erzielt werden.

## Patentansprüche

1. Mittel zum Aufhellen von keratinischen Fasern, **dadurch gekennzeichnet, dass** es in einem kosmetischen Träger
(i) mindestens ein kationisches Acylpyridiniumderivat der Formel (I), 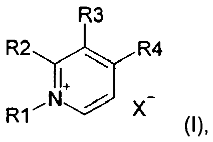 worin
R1 für eine C₁-C₆-Alkylgruppe, eine C₂-C₆-Alkenylgruppe, eine C₂-C₆-Hydroxy-alkylgruppe, eine C₁-C₆-Alkoxy-C₂-C₆-alkylgruppe, eine Carboxy-C₂-C₆-alkylgruppe, eine Aryl-C₁-C₆-alkylgruppe, eine Heteroaryl-C₁-C₆-alkylgruppe, eine Arylgruppe oder eine Heteroarylgruppe steht,
R2, R3 und R4 jeweils unabhängig voneinander für Wasserstoff, eine C₁-C₆-Alkylgruppe, ein Halogenatom oder eine C₁-C₆-Acylgruppe steht, mit der Massgabe, dass zumindest einer der Reste R2, R3 und R4 für eine C₁-C₆-Acylgruppe steht,
X⁻ für ein physiologisch verträgliches Anion steht,
(ii) mindestens 5 Gew.-%, bezogen auf das Gesamtgewicht des Mittels, Paraffinöl
und
(iii) als Oxidationsmittel mindestens Wasserstoffperoxid enthält.

2. Mittel nach Anspruch 1, **dadurch gekennzeichnet, dass** mindestens eine Verbindung der Formel (I) enthalten ist, die ausgewählt wird aus mindestens einer Verbindung der Gruppe, die gebildet wird aus 4-Acetyl-1-methylpyridinium-p-toluolsulfonat, 4-Acetyl-1-methylpyridinium-benzolsulfonat, 4-Acetyl-1-methylpyridiniumhydrogensulfat, 4-Acetyl-1-methyl-pyridiniumacetat, 4-Acetyl-1-allylpyridinium-p-toluolsulfonat, 4-Acetyl-1-allylpyridinium-benzolsulfonat, 4-Acetyl-1-allylpyridiniumhydrogensulfat, 4-Acetyl-1-allylpyridiniumacetat, 2-Acetyl-1-methylpyridinium-p-toluolsulfonat, 2-Acetyl-1-methylpyridinium-benzolsulfonat, 2-Acetyl-1-methylpyridiniumhydrogensulfat, 2-Acetyl-1-methylpyridiniumacetat, 2-Acetyl-1-allylpyridinium-p-toluolsulfonat, 2-Acetyl-1-allylpyridinium-benzolsulfonat, 2-Acetyl-1-allyl-pyridiniumhydrogensulfat und 2-Acetyl-1-allylpyridiniumacetat, insbesondere 4-Acetyl-1-methylpyridinium-p-toluolsulfonat.

3. Mittel nach einem der Ansprüche 1 bis 2, **dadurch gekennzeichnet, dass** die Acylpyridiniumderivate der Formel (I) in einer Menge von 0,1 bis 10 Gew.-%, insbesondere von 0,2 bis 4 Gew.-%. jeweils bezogen das Gesamtgewicht des Mittels, enthalten sind.

4. Mittel nach einem der Ansprüche 1 bis 3, **dadurch gekennzeichnet, dass** es von 10 bis 40 Gew.-%, insbesondere von 15 bis 30 Gew.-%, bezogen auf das Gesamtgewicht des Mittels, Paraffinöl enthält.

5. Mittel nach einem der Ansprüche 1 bis 4, **dadurch gekennzeichnet, dass** das Mittel einen pH-Wert von pH 2 bis pH 6, bevorzugt vom pH 2,5 bis pH 4,5 besitzt.

6. Verfahren zur Herstellung eines anwendungsbereiten Mittels zum Aufhellen von keratinischen Fasern, insbesondere menschlichen Haaren, **dadurch gekennzeichnet, dass** unmittelbar vor Anwendung ein Mittel M1 gemäß einem der Ansprüche 1 bis 5 und ein Mittel M2, enthaltend in einem kosmetisch verträglichen Träger mindestens ein Alkalisierungsmittel, miteinander vermischt werden und dass das anwendungsbereite Mittel einen pH-Wert von pH 6 bis pH 12, bevorzugt pH 9 bis pH 11 besitzt.

7. Verfahren nach Anspruch 6, **dadurch gekennzeichnet, dass** das Mittel M2 als Alkalisierungsmittel Ammoniak enthält.

8. Mehrkomponentenverpackungseinheit (Kit-of-Parts), umfassend mindestens zwei getrennt voneinander konfektionierte Container, wobei ein erster Container C1 mindestens ein Mittel M1 nach der Ansprüche 1 bis 5 und ein zweiter Container C2 mindestens ein Mittel M2, enthaltend in einem kosmetisch verträglichen Träger mindestens ein Alkalisierungsmittel, beinhaltet.

9. Kosmetische Verwendung eines anwendungsbereiten Mittels, das nach dem Verfahren nach einem der Ansprüche 6 oder 7 erhalten wurde, zum Aufhellen von keratinhaltigen Fasern, insbesondere menschlichen Haaren.

## Claims

1. Agent for lightening keratinic fibres, **characterised in that** it comprises in a cosmetic carrier
(i) at least one cationic acyl pyridinium derivative of the Formula (I), 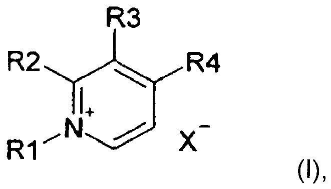 in which
R1 stands for a C₁-C₆ alkyl group, a C₂-C₆ alkenyl group, a C₂-C₆ hydroxyalkyl group, a C₁-C₆ alkoxy C₂-C₆ alkyl group, a carboxy C₂-C₆ alkyl group, an aryl C₁-C₆ alkyl group, a heteroaryl C₁-C₆ alkyl group, an aryl group or a heteroaryl group,
R2, R3 and R4 each independently of one another stands for hydrogen, a C₁-C₆ alkyl group, a halogen atom or a C₁-C₆ acyl group, with the proviso that at least one of the R2, R3 and R4 residues stands for a C₁-C₆ acyl group,
X⁻ stands for a physiologically acceptable anion,
(ii) at least 5 wt % paraffin oil, based on the total weight of the agent
and
(iii) at least hydrogen peroxide as the oxidising agent.

2. Agent according to claim 1, **characterised in that** at least one compound of Formula (I) is comprised, which is selected from at least one compound of the group consisting of 4-acetyl-1-methylpyridinium *p*-toluene sulfonate, 4-acetyl-1-methylpyridinium benzene sulfonate, 4-acetyl-1-methylpyridinium hydrogen sulfate, 4-acetyl-1-methylpyridinium acetate, 4-acetyl-1-allylpyridinium *p*-toluene sulfonate, 4-acetyl-1-allylpyridinium benzene sulfonate, 4-acetyl-1-allylpyridinium hydrogen sulfate, 4-acetyl-1-allylpyridinium acetate, 2-acetyl-1-methylpyridinium *p*-toluene sulfonate, 2-acetyl-1-methylpyridinium benzene sulfonate, 2-acetyl-1-methylpyridinium hydrogen sulfate, 2-acetyl-1-methylpyridinium acetate, 2-acetyl-1-allylpyridinium *p*-toluene sulfonate, 2-acetyl-1-allylpyridinium benzene sulfonate, 2-acetyl-1-allylpyridinium hydrogen sulfate and 2-acetyl-1-allylpyridinium acetate, in particular 4-acetyl-1-methylpyridinium *p*-toluene sulfonate.

3. Agent according to one of claims 1 to 2, **characterised in that** the acyl pyridinium derivatives of the Formula (I) are comprised in an amount of 0.1 to 10 wt %, particularly 0.2 to 4 wt %, each based on the total weight of the agent.

4. Agent according to one of claims 1 to 3, **characterised in that** it comprises from 10 to 40 wt %, in particular 15 to 30 wt % paraffin oil, based on the total weight of the agent.

5. Agent according to one of claims 1 to 4, **characterised in that** the pH of the agent is from pH 2 to pH 6, preferably pH 2.5 to pH 4.5.

6. Process for preparing a ready-for-use agent for lightening keratinic fibres, particularly human hair, **characterised in that** an agent M1 according to one of claims 1 to 5 and an agent M2, comprising in a cosmetically acceptable carrier at least one alkalising agent, are mixed together directly prior to use and that the ready-for-use agent exhibits a pH of pH 6 to pH 12, preferably pH 9 to pH 11.

7. Process according to claim 6, **characterised in that** the agent M2 comprises ammonia as the alkalising agent.

8. Kit of parts comprising at least two containers assembled separately from one another, wherein a first container C1 contains at least one agent M1 according to claims 1 to 5 and a second container C2 contains at least one agent M2, comprising at least one alkalising agent in a cosmetically acceptable carrier.

9. Cosmetic use of a ready for use agent that was obtained according to the process according to one of claims 6 or 7 for lightening keratin-containing fibres, particularly human hair.

## Revendications

1. Agent pour l'éclaircissement de fibres kératiniques, **caractérisé en ce qu'**il contient, dans un support cosmétique,
(i) au moins un dérivé cationique d'acylpyridinium de formule (I) 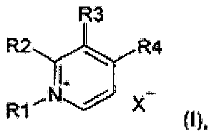 où
R1 représente un groupe C₁-C₆-alkyle, C₂-C₆-alcényle, C₂-C₆-hydroxyalkyle, C₁-C₆-alcoxy-C₂-C₆-alkyle, carboxy-C₂-C₆-alkyle, aryl-C₁-C₆-alkyle, hétéroaryl-C₁-C₆-alkyle, aryle ou hétéroaryle,
R2, R3 et R4 représentent, à chaque fois indépendamment les uns des autres, hydrogène, un groupe C₁-C₆-alkyle, un atome d'halogène ou un groupe C₁-C₆-acyle, à condition qu'au moins un des résidus R2, R3 et R4 représente un groupe C₁-C₆-acyle,
X⁻ représente un anion physiologiquement acceptable,
(ii) au moins 5% en poids, par rapport au poids total de l'agent, d'huile de paraffine
et
(iii) comme oxydant au moins du peroxyde d'hydrogène.

2. Agent selon la revendication 1, **caractérisé en ce qu'**il contient au moins un composé de formule (I), qui est choisi parmi au moins un composé du groupe formé par le p-toluènesulfonate de 4-acétyl-1-méthylpyridinium, le benzènesulfonate de 4-acétyl-1-méthylpyridinium, l'hydrogénosulfate de 4-acétyl-1-méthylpyridinium, l'acétate de 4-acétyl-1-méthylpyridinium, le p-toluènesulfonate de 4-acétyl-1-allylpyridinium, le benzènesulfonate de 4-acétyl-1-allylpyridinium, l'hydrogénosulfate de 4-acétyl-1-allylpyridinium, l'acétate de 4-acétyl-1-allylpyridinium, le p-toluènesulfonate de 2-acétyl-1-méthylpyridinium, le benzènesulfonate de 2-acétyl-1-méthylpyridinium, l'hydrogénosulfate de 2-acétyl-1-méthylpyridinium, l'acétate de 2-acétyl-1-méthylpyridinium, le p-toluènesulfonate de 2-acétyl-1-allylpyridinium, le benzènesulfonate de 2-acétyl-1-allylpyridinium, l'hydrogénosulfate de 2-acétyl-1-allylpyridinium et l'acétate de 2-acétyl-1-allylpyridinium, en particulier le p-toluènesulfonate de 4-acétyl-1-méthylpyridinium.

3. Agent selon l'une quelconque des revendications 1 à 2, **caractérisé en ce que** les dérivés d'acylpyridinium de formule (I) sont contenus en une quantité de 0,1 à 10% en poids, en particulier de 0,2 à 4% en poids, à chaque fois par rapport au poids total de l'agent.

4. Agent selon l'une quelconque des revendications 1 à 3, **caractérisé en ce qu'**il contient 10 à 40% en poids, en particulier 15 à 30% en poids, par rapport au poids total de l'agent, d'huile de paraffine.

5. Agent selon l'une quelconque des revendications 1 à 4, **caractérisé en ce que** l'agent présente un pH de pH 2 à pH 6, de préférence de pH 2,5 à pH 4,5.

6. Procédé pour la préparation d'un agent prêt à l'emploi pour l'éclaircissement de fibres kératiniques, en particulier de cheveux humains, **caractérisé en ce qu'**on mélange l'un avec l'autre, juste avant l'utilisation, un agent M1 selon l'une quelconque des revendications 1 à 5 et un agent M2 contenant, dans un support cosmétiquement acceptable, au moins un agent d'alcalinisation et **en ce que** l'agent prêt à l'emploi présente un pH de pH 6 à pH 12, de préférence de pH 9 à pH 11.

7. Procédé selon la revendication 6, **caractérisé en ce que** l'agent M2 contient de l'ammoniaque comme agent d'alcalinisation.

8. Unité d'emballage à plusieurs composants (Kit-of-Parts), comprenant au moins deux récipients confectionnés séparément l'un de l'autre, un premier récipient C1 contenant au moins un agent M1 selon les revendications 1 à 5 et un deuxième récipient C2 contenant au moins un agent M2 contenant, dans un support cosmétiquement acceptable, au moins un agent d'alcalinisation.

9. Utilisation cosmétique d'un agent prêt à l'emploi, qui a été obtenu selon le procédé selon l'une quelconque des revendications 6 ou 7 pour l'éclaircissement de fibres kératiniques, en particulier de cheveux humains.
